(19) Europäisches Patentamt
European Patent Office
Office européen des brevets

(11) Publication number: **0 196 786 A2**

# (12) EUROPEAN PATENT APPLICATION

(21) Application number: 86301460.1

(22) Date of filing: 28.02.86

(51) Int. Cl.[4]: C07D 405/12 , A01N 47/36

(30) Priority: 28.02.85 US 706774
27.01.86 US 821323

(43) Date of publication of application: 08.10.86 Bulletin 86/41

(84) Designated Contracting States: AT BE CH DE FR GB IT LI LU NL SE

(71) Applicant: E.I. DU PONT DE NEMOURS AND COMPANY
1007 Market Street
Wilmington Delaware 19898(US)

(72) Inventor: Wolf, Anthony David
151 Kirkcaldy Drive
Elkton Maryland 21921(US)

(74) Representative: Hildyard, Edward Martin et al
Frank B. Dehn & Co. Imperial House 15-19 Kingsway
London WC2B 6UZ(GB)

## (54) Herbicidal sulfonamides.

(57) Compounds of the formula:

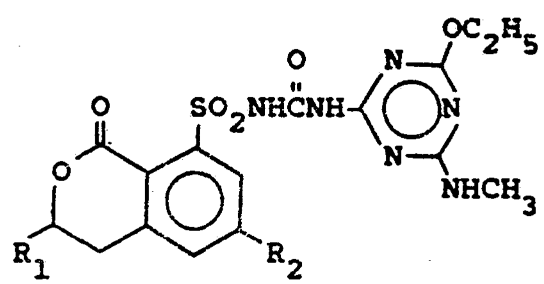

wherein

$R_1$ is H or $CH_3$; and

$R_2$ is H, F, Cl or $CH_3$;

exhibit herbicidal activity, especially selective activity on rape. The compounds may be made e.g. by reacting the appropriate arylsulfonyl isocyanate with the appropriate aminotriazine.

EP 0 196 786 A2

# HERBICIDAL SULFONAMIDES

## Background of the Invention

This application relates to specific sulfonamides which have high activity on grasses and broadleaves and are simultaneously safre on rape.

Compounds of Formula I below are generically disclosed in EP-A-107,979 which claims, in part, herbicidal sulfonamides of formula

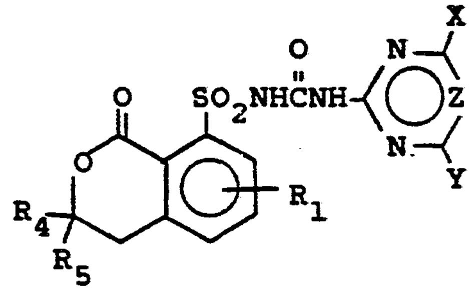

I

wherein $R_1$ is H, F, Cl, Br, $CH_3$, $OCH_3$ $CF_3$, $SCH_3$ or $OCF_2H$;

$R_4$ is H, $C_1$-$C_4$ alkyl, Cl or Br;

$R_5$ is H or $CH_3$;

X is $CH_3$, $OCH_3$, OCHG$_2$CH3, Cl, F, BR, $OCF_2H$, $CH_2F$ or $CF_3$;

Y is H, $CH_3$, $OCH_3$, $OC_2H_5$, $CH_2OCH_3$, $NH_2$, $NHCH_3$, N-$(CH_3)_2$, $C_2H_5$, $CF_3$, $SCH_3$, $OCH_2CH=CH_2$, $OCH_2C\equiv CH$, $OCH_2CF_3$, CN, $N_3$, $OCH_2CH_2OCH_3$, $CH_2SCH_3$, $CR_6(QCH_3)_2$,

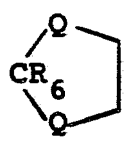 , 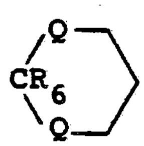 , 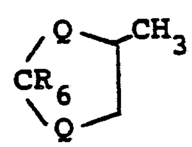 ,

$CR_6(QCH_2CH_3)_2$ or $QFC_2T$ where

Q is O or S and T is H, CHClF, CHBrF or $CHFCF_3$;

and Z is CH, N, $CCH_3$, $CC_2H_5$ CCl or CBr.

Compounds of Formula I are also generically disclosed in South African Patent Application 83/5165 (Swiss priority 7/16/82, published 1/16/84) which discloses herbicidal sulfonamides of formula

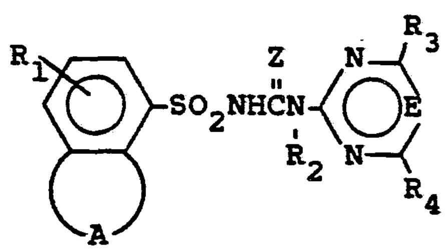

wherein

A is an unsubstituted or substituted bridge of 3 or 4 atoms which contains 1 or 2 oxygen, sulfur or nitrogen atoms and, together with the linking carbon atom, forms a non-aromatic 5-or 6-membered heterocyclic ring system, with the proviso that two oxygen atoms are separated by at least one carbon atom and that oxygen and sulfur atoms are only linked to each other if the sulfur atom takes the form of the -SO-or $SO_2$-group, and $R_2$ is hydrogen, halogen, nitro, $C_1$-$C_4$ alkyl, $C_1$-$C_4$ haloalkyl, $C_1$-$C_4$ alkoxy, $C_1$-$C_2$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_1$ is H, halogen, $NO_2$, $C_1$-$C_4$ alkyl, $C_1$-$C_4$ haloalkyl, $C_1$-$C_4$ alkoxy, $C_1$-$C_4$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy;

$R_3$ and $R_4$, each independently of the other, are hydrogen, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxy-alkoxy or $NR_5R_6$, wherein $R_5$ and $R_6$ are hydrogen or $C_1$-$C_4$ alkyl; and E is CH or N.

Summary of the Invention

This invention relates to novel compounds of Formula I, agriculturally suitable compositions containing them, and their method-of-use as preemergent or postemergent herbicides or as plant growth regulants.

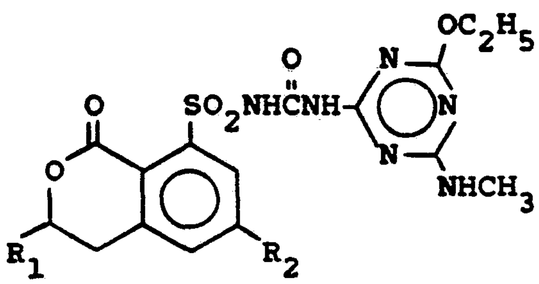

I

wherein $R_1$ is H or $CH_3$; and

$R_2$ is H, F, Cl or $CH_3$.

Specifically Preferred for reasons of their highest herbicidal activity, greatest plant growth regulant activity and/or most favorable ease of synthesis are:

- N-[[4-ethoxy-6-(methylamino)-1,3,5-triazin-2-yl]-aminocarbonyl]-3,4-dihydro-3-methyl-1-oxo-1H-2-benzopyran-8-sulfonamide, m.p. 223-224°C(d); and

- N-[[4-ethoxy-6-(methylamino)-1,3,5-triazin-2-yl]-aminocarbonyl]-3,4-dihydro-1-oxo-1H-2-benzopyran-8-sulfonamide, m.p. 220-225°C(d).

DETAILED DESCRIPTION OF THE INVENTION

The compounds of Formula I can be prepared by one or more of the methods described below in Equations 1, 2 and 3.Preferred reaction conditions are given by way of illustration.

As shown in Equation 1, compounds of Formula I can be prepared by reacting a sulfonylisocyanate of Formula II with an appropriate heterocyclic amine of Formula III, where $R_1$ = H or $CH_3$ and $R_2$ = H, F, Cl, or $CH_3$.

Equation 1

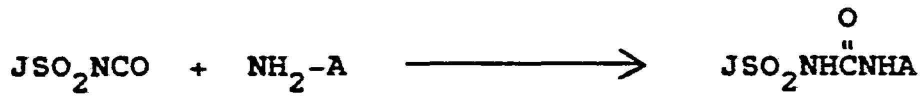

II III I

where J is 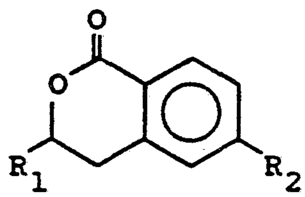 ; and

A is 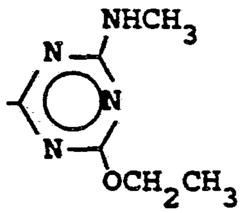

The reaction is carried out at 25° to 100°C in an inert, aprotic solvent such as methylene chloride or acetonitrile for 0.5 to 24 hours as taught in U.S. Patent 4,127,405.

Alternatively, compounds of Formula I can be prepared by reacting a sulfonylcarbamate of Formula IV with an appropriate amine of Formula III, as shown in Equation 2.

Equation 2

$JSO_2NHCO_2Ph$ + III ⟶ I

IV

where J is as defined in Equation 1.

The reaction is carried out at 50° to 100°C in a solvent such as dioxane for 0.5 to 24 hours as taught in EPO Publication No. 44,807. The required carbamates IV are prepared by reacting the corresponding sulfonamides V with diphenylcarbonate in the presence of a strong base.

Alternatively, compounds of Formula I may be prepared as shown in Equation 3, by the reaction of a sulfonamide V with the appropriate carbamate heterocycle, VII.

Equation 3

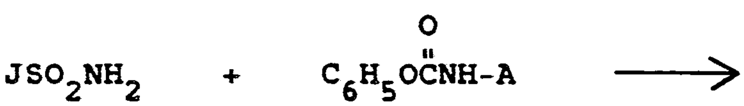

V VII I

where A and J are as defined in Equation 1.

The reaction of Equation 3 is best carried out according to the methods taught in EPO Publication No. 70,804.

The intermediate sulfonylisocyanates of Formula II can be prepared by the two-step procedure shown below in Equation 4, starting from the appropriate sulfonamides, V.

Equation 4

a) V + $SOCl_2$ ⟶ $JSO_2NSO$ VIII

b) VIII + $COCl_2$ ⟶ IIwhere J is as defined in Equation 1.

The reactions of Equation 4a and 4b are best carried out according to the procedure of Ulrich, et al. as described in J. Org.Chem., 34, 3200 (1969). The sulfonamide V is boiled under reflux with an excess of thionyl chloride which functions as both a reactant and solvent. When the sulfonamide protons are no longer detectable by proton NMR - (15-20 hrs. on the average), the thionyl chloride is removed under reduced pressure and the residue is dissolved in an inert solvent such as toluene, benzene, xylenes, etc. A catalytic amount of pyridine is added. The mixture is treated with at least one equivalent of phosgene and heated to

60°-140°C with 80°-100°C preferred. Conversion to the isocyanate is substantially complete within about 1/4 to 3 hours. The mixture containing the sulfonyl isocyanate can be used directly or the sulfonyl isocyanate can be isolated in pure form by filtration and evaporation of the filtrate followed by vacuum distillation if necessary.

The sulfonyl isocyanates II can also be prepared from the sulfonamides V by a two step procedure involving (a) reacting the sulfonamides with n-butyl isocyanate in the presence of a base such as $K_2CO_3$. at reflux in an inert solvent such as 2-butanone or acetonitrile, forming a n-butyl sulfonylurea; and (b) reacting this compound with phosgene and a tertiary amine catalyst at reflux in xylene solvent. This method is similar to a procedure taught by Ulrich and Sayigh, Newer Methods of Preparative OrganicChemistry, Vol. VI, p. 223-241, Academic Press, New York and London, W. Forest Ed.

The sulfonamides of Formula V may be prepared as shown in Equation 5.

Equation 5

(a)

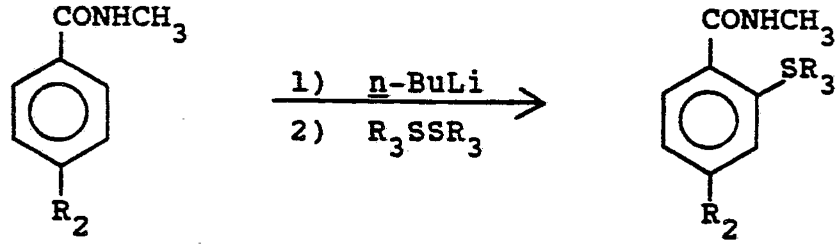


VIII IX

(b)

IX

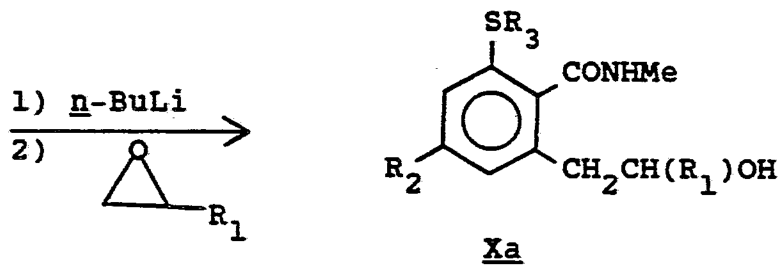


Xa

+

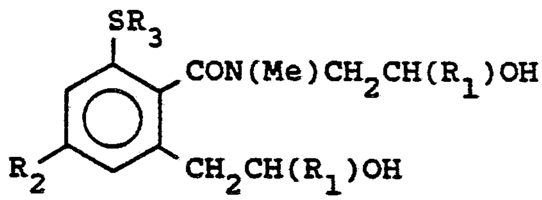

Xb

(c)

Xa + Xb

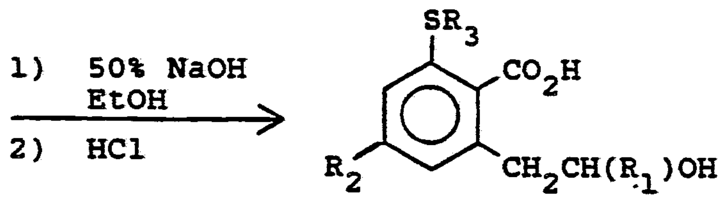


+ $J-SR_3$

XI XII

(d) XI + XII

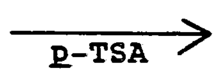


XII

(e) XII $\xrightarrow{Cl_2}$ $J-SO_2Cl$

XIII

(f) XIII $\xrightarrow{NH_3}$ V

where J is as defined in Equation 1;

$R_1$ is H or $CH_3$;

$R_2$ is H, $CH_3$, F or Cl;

$R_3$ is $CH_2CH_3$ or $CH_2CH_2CH_3$.

The reaction of Equation 5a, in which lithiated benzamides are alkylated with disulfides to provide the corresponding sulfides, such as IX, is most conveniently carried out by the procedures described by J. J. Fitt and H. W. Gschwend, J. Org. Chem., 41, 4029 (1976).

Reaction of lithiated benzamides of Formula IX with propylene oxide to provide the corresponding alcohols X, as shown in Equation 5b, can be accomplished according to the method taught by N. S. Narashimhan and B. H. Bhide, Chem. Commun., 1970, 1552; also N. S. Narashimhan and B. H. Bhide, Tetrahedron, 27, 6171 (1971).

The hydrolysis and cyclization of alcohol amides, such as X, to the corresponding benzopyrans XII, as shown in Equations 5c and 5d, is also taught by N. S. Narashimhan and B. H. Bhide, Chem. Commun., 1970, 1552.

The reaction of Equation 5f, the oxidative chlorination of sulfides, such as XII, to provide the corresponding sulfonyl chlorides XIII, is carried out by the addition of molecular chlorine or a chlorine equivalent to the sulfide in the presence of water at 15° to 80°C in an aliphatic carboxylic acid solvent such as acetic acid or in an inert organic solvent such as dichloromethane for 1 to 24 hours.

As shown in Equation 5f, sulfonamides of Formula V can be prepared from the corresponding sulfonyl chlorides of Formula XIII by contacting with either anhydrous or aqueous ammonia. The preparation of sulfonamides from sulfonyl chlorides is widely reported in the literature, for reviews see: F. Hawking and J. S. Lawrence, "The Sulfonamides", H. D. Lewis and Co., London, 1950 and E. H. Northey, "The Sulfonamides and Allied Compounds", Reinhold Publishing Corp., New York 1948.

Some of the sulfides of Formula IX are more conveniently prepared by the displacement of chlorine from the ortho-chloro substituted benzamides XIV with the salt of an alkyl mercaptan as shown in Equation 6..

Equation 6

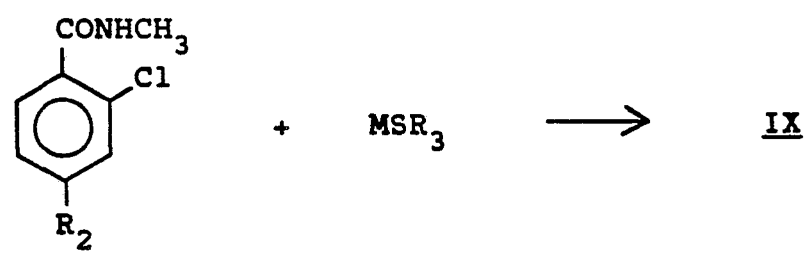

XIV

where M = Na or K

$R_2$ and $R_3$ are as defined in Equation 5.

The reaction of Equation 6 can be carried out at 25° to 80°C in a polar solvent such as N,N-dimethyl formamide. The displacement of halides by the metal salts of mercaptans is well known in the literature, see J. R. Campbell, J. Org. Chem., 27, 1830 (1964).

The aminotriazine III is known in the art. For example, see J. Amer. Chem. Soc., 71, 3248 (1949).

Example 1

3,4-Dihydro-8-(ethylthio)-3-methyl-1-oxo-1H2-benzopyran

A solution of 70.0 g 2-ethylthio-N-methyl-benzamide in 875 ml of dry tetrahydrofuran, was cooled to -45°C and 467 ml of 1.6 M n-butyllithium added dropwise at -45°C. After stirring 2 hours at -45°C, the orange solution was allowed to warm to 0°C and 76 ml of propylene oxide were added dropwise. The reaction was allowed to slowly warm to room temperature. After 1 hour at room temperature, the mixture was poured into saturated ammonium chloride and sodium chloride added until the solution was saturated. The product was extracted with ether, washed with brine and dried over sodium sulfate. Concentration gave a mixture of 2-(ethylthio)-6(2-hydroxypropyl)-N-methyl-benzamide and 2-(ethylthio)-6-(2-hydroxypropyl)-N-(2-hydroxypropyl)-N-methyl-benzamide.

A solution of the above mixture of benzamides, 500 ml ethanol, 100 ml of 50% sodium hydroxide and 100 ml water was refluxed for 16 hours. The resulting dark brown solution was stirred at room temperature an additional 36 hours, poured into 500 ml water and washed two times with ether. The aqueous phase was acidified with 10% HCl and sodium chloride was added until the aqueous phase was saturated. The product was extracted with methylene chloride and dried over sodium sulfate. Concentration gave a mixture of 2-(ethylthio)-6-(2-hydroxypropyl)benzoic acid and 3,4-dihydro-8-(ethylthio)-3-methyl-1-oxo-1H-2-benzopyran.

A solution of the above mixture of 2-(ethylthio)-6-(2-hydroxypropyl)-benzoic acid and 3,4-dihydro-8-(ethylthio)-3-methyl-1-oxo-1H-2-benzopyran, 200 ml toluene and 6.8 g of p-toluenesulfonic acid was heated at reflux with the azeotropic removal of water (5.5 ml) for 2 hours. Concentration gave 62.7 g of 3,4-dihydro-8-(ethylthio)-3-methyl-1-oxo-1H-2-benzopyran as a brown oil, IR (neat) 1725 (C=O) $cm^{-1}$.

NMR($CDCl_3$) δ: 1.34-1.49 (6H, m);

2.85-2.93 (4H, m);

4.45-4.71 (1H, m);

6.85 (1H, d);

7.20-7.40 (2H, m).

Example 2

3,4-Dihydro-3-methyl-1-oxo-1H-2-benzopyran-8sulfonyl chloride

A solution of 62.7 g of the product from Example 1, 700 ml acetic acid and 12.7 ml of water was cooled to 15°C and 42.6 ml of chlorine added dropwise at 15° to 20°C. After the addition was complete, the reaction was allowed to warm to room temperature. After 30 minutes at room temperature, the mixture was poured into ice water and the resulting precipitate collected by filtration, providing 55.7 g of a yellow solid, m.p. 145-149°C. IR (Nujol) 1730 (C=0), $cm^{-1}$.

NMR (DMSO) δ: 1.36 (3H, d);

2.70-3.00 (2H, m);

4.35-4.60 (1H, m);

7.32 (1H, d);

7.46 (1H, t);

7.86 (1H, d).

Example 3

3,4-Dihydro-3-1-oxo-1H-2-benzopyran-8-sulfonamide

A solution of 40 g of the product from Example 2, 800 ml of methylene chloride and 200 ml of tetrahydrofuran was cooled to -45°C. Anhydrous ammonia was added dropwise at -45°C. The resulting suspension was allowed to warm to room temperature. After 30 minutes, the mixture was poured into 10% HCl. An insoluble precipitate remained and was collected by filtration to afford 22.3 g of the title compound as a white solid, m.p. 196-204°C. The organic phase was dried ($Na_2SO_4$) and concentrated to provide an additional 10.7 g of 3,4-dihydro-3-methyl-1-oxo-1H-2-benzopyran-8-sulfonamide, m.p. 199-204°C. IR (Nujol) 3230 and 3320 ($NH_2$), 1700 (C=0) $cm^{-1}$.

NMR (DMSO) δ: 1.38 (3H, d);

2.85-3.15 (2H, m);

4.60-4.75 (1H, m);

7.23 (1H, s);

7.65 (1H, d);

7.76 (1H, t);

7.97 (1H, d).

Example 4

3,4-Dihydro-3-methyl-1-oxo-1H-2-benzopyran-8-sulfonyl isocyanate

A suspension of 20 g of the product from Example 3 and 300 ml of thionyl chloride was refluxed for 48 hours. The resulting solution was then allowed to cool and excess thionyl chloride was removed in vacuo. The residue was dissolved in 70 ml toluene and again concentrated. To the residue was added 30 drops of dry pyridine and 500 ml toluene. the mixture was heated to 70°C and 20 ml of phosgene added in one portion. The reaction mixture was heated at 100°C for 2 hours, the cold trap removed and the reaction mixture kept at 100°C for 30 minutes. The reaction mixture was allowed to cool, and all volatile materials removed in vacuo. The desired sulfonyl isocyanate was obtained as a yellow solid. IR (Nujol) 2238 (NCO) and 1720 (C=0) $cm^{-1}$.

Example 5

N-[(4-Ethoxy-6-(methylamino)-1,3,5-triazin-2-yl)-aminocarbonyl]-3,4-Dihydro-3-methyl-1-oxo-1H-2-benzopyran-8-sulfonamide

A suspension of 1.06 g of the product from Example 4, 10 ml methylene chloride and 0.5 g of 2-amino-4-ethoxy-6-(methylamino)-1,3,5-triazine was stirred at room temperature. After approximately 5 minutes all solids were dissolved, and a new precipitate formed after 1 hour. The solids were collected by filtration and washed with n-butyl chloride to provide 0.78 g of the title compound, m.p. 223-224°C (d). IR (Nujol) 1713 and 1727 (C=0) $cm^{-1}$.

NMR (DMSO + $CDCl_3$) δ: 1.30 (3H, t);

1.45 (3H, d);

2.90 (3H, d, $NHCH_3$);

2.90 (2H, m);

4.00-4.90 (3H, m);

7.50-8.30 (3H, m);

10.40 (1H, br s, NH);

12.35 (1H, br s, NH).

Using the methods described herein and the procedures of Examples 1 to 5, the compounds of Table I may be prepared.

TABLE I

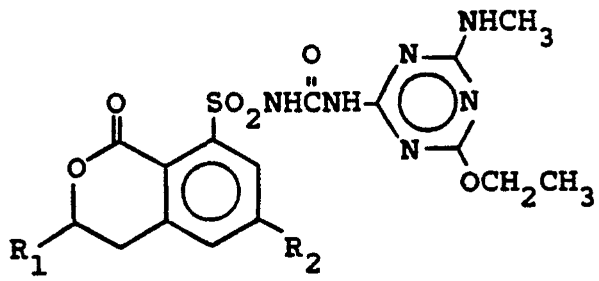

| $R_1$ | $R_2$ | m.p. (°C) |
|---|---|---|
| $CH_3$ | H | 223-224 |
| H | H | 220-225 |
| $CH_3$ | F | |
| H | F | |
| $CH_3$ | Cl | 220-224 |
| H | Cl | |
| $CH_3$ | $CH_3$ | |
| H | $CH_3$ | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table II

| | Active Ingredient | Weight Percent* Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 6

Wettable Powder

| | |
|---|---|
| N-[[4-ethoxy-6-(methylamino)-1,3,5-triazin-2-yl]aminocarbonyl]-3,4-dihydro-3-methyl-1-oxo-1H-2-benzopyran-8-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended and ground in a hammer-mill to produce particles with an average particle size of less than 25 microns in diameter. The material is reblended and sifted through a U.S.S. No. 50 sieve (0.33 mm opening) before being packaged.

Example 7

Wettable Powder

| | |
|---|---|
| N-[[4-ethoxy-6-(methylamino)-1,3,5-triazin-2-yl]aminocarbonyl]-3,4-dihydro-3-methyl-1-oxo-1H-2-benzopyran-8-sulfonamide | 65% |
| dodecylphenol polyethyelen glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammermill to produce particles essentially all below 100 microns, the material is reblended and sifted through a U.S.S. No. 50 sieve (0.3 mm opening) and packaged.

Example 8

Granule

| | |
|---|---|
| Wettable Powder of Example 6 | 15% |
| gypsum | 69% |
| potassium sulfate | 16% |

The ingredients are blended in a rotating mixer and water sprayed on to accomplish granulation. When most of the material has reached the desired range of 1.0 to 0.42 mm (U.S.S. No. 18 to 40 sieves), the granules are removed, dried, and screened. Oversize material is crushed to produce additional material in the desired range. These granules contain 12% active ingredient.

Example 9

Extruded Pellet

| | |
|---|---|
| N-[[4-ethoxy-6-(methylamino)-1,3,5-triazin-2-yl]aminocarbonyl]-3,4-dihydro-1-oxo-1H-2-benzopyran-8-sulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammermilled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 10

Wettable Powder

| | |
|---|---|
| N-[[4-ethoxy-6-(methylamino)-1,3,5-triazin-2-yl]aminocarbonyl]-3,4-dihydro-3-methyl-1-oxo-1H-2-benzopyran-8-sulfonamide | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended and sifted through a U.S.S. No. 50 sieve (0.3 mm opening) before packaging.

Example 11

Oil Suspension

| | |
|---|---|
| N-[[4-ethoxy-6-(methylamino)-1,3,5-triazin-2-yl]aminocarbonyl]-3,4-dihydro-1-oxo-1H-2-benzopyran-8-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 12

Wettable Powder

| | |
|---|---|
| N-[[4-ethoxy-6-(methylamino)-1,3,5-triazin-2-yl]aminocarbonyl]-3,4-dihydro-3-methyl-1-oxo-1H-2-benzopyran-8-sulfonamide | 50% |
| sodium alkylnaphthalenesulfonate | 2% |
| low viscosity methyl cellulose | 2% |
| diatomaceous earth | 46% |

The ingredients are blended, coarsely hammermilled and then air milled to produce particles of active essentially all below 10 microns in diameter. The product is reblended before packaging.

Example 13

High Strength Concentrate

| | |
|---|---|
| N-[[4-ethoxy-6-(methylamino)-1,3,5-triazin-2-yl]aminocarbonyl]-3,4-dihydro-1-oxo-1H-2-benzopyran-8-sulfonamide | 98.5% |
| silica aerogel | 0.5% |
| synthetic amorphous silica | 1.0% |

The ingredients are blended and ground in a hammermill to produce a high strength concentrate essentially all passing a U.S.S. No. 50 screen (0.3 mm openings). This material may then be formulated in a variety of ways.

Example 14

Oil Suspension

| | |
|---|---|
| N-[[4-ethoxy-6-(methylamino)-1,3,5-triazin-2-yl]aminocarbonyl]-3,4-dihydro-1-oxo-1H-2-benzopyran-8-sulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 15

Aqueous Suspension

| | |
|---|---|
| N-[[4-ethoxy-6-(methylamino)-1,3,5-triazin-2-yl]aminocarbonyl]-3,4-dihydro-3-methyl-1-oxo-1H-2-benzopyran-8-sulfonamide | 25% |
| hydrated attapulgite | 3% |
| crude calcium ligninsulfonate | 10% |
| sodium dihydrogen phosphate | 0.5% |
| water | 61.5% |

The ingredients are ground together in a ball or roller mill until the solid particles have been reduced to diameters under 10 microns.

Utility

The compounds are selective herbicides for use in oilseed rape (Brassica napus and Brassicacampestris). Control of broadleaf and grassy weeds can be expected at rates between 16 and 250 g/ha. A particularly significant feature of these compounds is the ability to control wild mustard (Brassica kaber), a species closely related to oilseed rape (Brassicasp.). The compounds can be applied as a preemergence or postemergence treatment and exhibits excellent safety to the rapeseed crop.

The compounds of this invention may be used in combination with other commercial herbicides. They are particularly useful in combination with the following herbicides:

| Common Name or Code Number | Chemical Name |
|---|---|
| alloxydim-sodium | Sodium salt of 2-(1-allyloxyaminobutylidene)-5,5-dimethyl-4-methoxycarbonylcyclohexane-1,3-dione |
| carbetamide | N-ethyl-2-[[(phenylamino)carbonyl]oxy]propanamide (R)-isomer |
| dalapon | 2,2-dichloropropionic acid |
| diallate | S-(2,3-Dichloroallyl)diisopropylthiocarbamate |
| diclofop methyl | Methyl 2-[4-(2',4'-dichlorophenoxy)phenoxy]propanate |
| diuron | 3-(3,4-dichlorophenyl)-1,1-dimethylurea |
| fluazifop-butyl | (±)-butyl 2-[4-[(5-(trifluoromethyl)-2-pyridinyl)oxy]-phenoxy]propanoate |
| haloxyfop-methyl | Methyl 2-(4-((3-chloro-5-(trifluoromethyl)-2-pyridinyl)oxy)-phenoxy)propanoate |

| Common Name or Code Number | Chemical Name |
|---|---|
| methazachlor | 2-chloro-N-(2,6-dimethylphenyl)-N-(1H-pyrazol-1-yl methyl)acetamide |
| napropamide | 2-(α-Naphthoxy)-N,N-diethyl propionamide |
| nitralin | 4-methylsulfonyl-2,6-dinitro-N,N-dipropylaniline |
| propyzamide | 3,5-dichloro(N-1,1-dimethyl)-2-propynyl)benzamide |
| sethoxydim | 2-[1-(ethoxyimino)butyl]-5-[2-(ethylthio)propyl]-3-hydroxy-2-cyclohexen-1-one |
| TCA | trichloroacetic acid |
| 3,6-dichloro-picolinic acid | 3,6-Dichloro-2-pyridinecarboxylic acid |
| trifluralin | α,α,α-Trifluoro-2,6-dinitro-N,N-dipropyl-p-toluidine |
| BAS 518 | 7-Chloro-3-methylquinoline-8-carboxylic acid |
| quinofop ethyl | ethyl 2-[4-[(6-chloro-2-quinoxalinyl)oxy]phenoxy]propionate |

The selective herbicidal properties of the subject compounds that make them useful as rape herbicides were discovered in a number of greenhouse tests, conducted as described below.

Test A

Seeds of the following crops and weed species were sown into 15 cm diameter pots containing Sassafras sandy loam soil: wheat (Triticum aestivum), barley ( Hordeum vilgare), rapeseed (Brassica napus), chickweed (Stellaria media), radish (Raphnus raphanistrum), pigweed (Amaranthus retroflexus), buckwheat ( Polygonum convolvulus), Galium (Galium aparine), mustard (Brassica kaber), lambsquarters (Chenopodiumalbum), Matricaria (Matricaria inodore), speedwell (Veronica persica), green foxtail ( Setaria viridis), annual ryegrass (Lolium multiforum), blackgrass (Alopecurus myosuroides), and wild oats (Avena fatua ). Compound A was formulated in a non-phytoxic solvent and applied prior to weed and crop emergence and when crops and weeds has grown to the 2-3 leaf stage - (post-emergence). The degree of crop injury and weed control was evaluated visually, 3 to 4 weeks after compound application using a scale of 0 to 100%, where 0 = no control or injury and 100 = complete death.

Results are summarized in Table 1. Data represent the results of two tests.

Compounds

Compound A

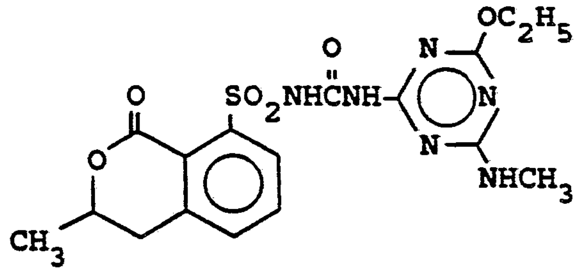

Compound B

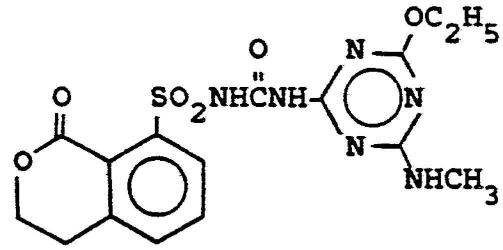

Compound C

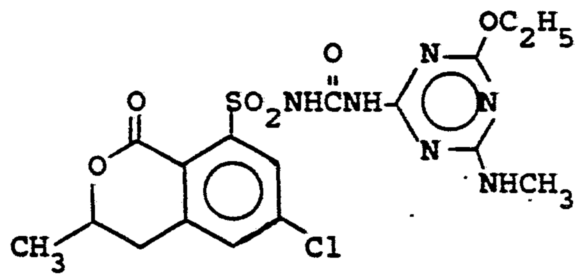

Table 1 Results of Test A

Compound A

| | Postemergence | | | | Preemergence | | | |
|---|---|---|---|---|---|---|---|---|
| Rate g/ha | 16[1] | 20 | 64 | 125 | 30 | 64 | 125 | 250 |
| French rape | 0 | 0 | 10 | 10 | 0 | 10 | 10 | 70 |
| Canada rape | 0 | 0 | 10 | 10 | 0 | 0 | 10 | 50 |
| Wheat | 50 | 70 | 85 | 100 | 0 | 95 | 95 | 100 |
| Barley | 25 | 75 | 80 | 100 | 0 | 70 | 85 | 95 |
| Wild Oats | 85 | 95 | 100 | 100 | 40 | 87 | 83 | 90 |
| Blackgrass | 95 | 95 | 100 | 100 | 85 | 95 | 90 | 100 |
| Ryegrass | 75 | 93 | 100 | 100 | 80 | 95 | 95 | 100 |
| Green foxtail | 35 | 75 | 100 | 100 | 90 | 97 | 93 | 100 |
| Matricaria | 0 | 50 | 80 | 100 | 75 | 93 | 95 | 100 |
| Speedwell | 0 | 10 | 20 | 90 | 20 | 12 | 68 | 85 |
| Galium | 0 | 85 | 97 | 100 | 0 | 80 | 83 | 95 |
| Chickweed | 25 | 75 | 97 | 100 | 25 | 60 | 90 | 100 |
| Pigweed | 80 | 95 | 100 | 100 | 0 | 93 | 100 | 100 |
| Mustard | 50 | 87 | 87 | 100 | 50 | 97 | 100 | 95 |
| Wild buckwheat | 0 | 45 | 45 | 90 | 20 | 40 | 83 | 90 |
| Lambsquarters | 75 | 85 | 90 | 100 | 25 | 97 | 100 | 100 |
| Wild radish | 0 | 0 | 50 | 20 | 0 | 43 | 63 | 85 |
| Smartweed | 0 | 87 | 20 | 80 | 0 | 93 | 93 | 90 |

1 Rates = grams per hectare

Test B

Postemergence

Two round pans (25 cm diameter by 12.5 cm deep) were filled with Sassafras sandy loam soil. One pan was planted with blackgrass (Alopecurus myosuroides), sugarbeets, nutsedge (Cyperus rotundus) tubers, rape (Brassica napus), crabgrass (Digitaria sanguinalis), sicklepod - (Cassiaobtusifolia), teaweed Sida Spinosajimsonweed - (Datura stramonium), velvetleaf (Abutilontheophrasti), and giant foxtail (Setaria faberii). The other pan was planted with wheat, cotton, rice, corn, soybean, wild oats (Avena fatua), cocklebur Xantiumpensylvanicum), morningglory (Ipomoea hederacea), johnsongrass (Sorghum halepense) and barnyardgrass (Echinochloacrusgalli). The plants were grown for approximately fourteen days, then sprayed postemergence with the chemicals dissolved in a non-phytotoxic solvent.

Preemergence

Two round pans (25 cm diameter by 12.5 cm deep) were filled with Sassafras sandy loam soil. One pan was planted with blackgrass, sugarbeets, nutsedge, rape, crabgrass, sicklepod, teaweed, jimsonweed, velvetleaf, and giant foxtail. The other pan was planted with wheat, cotton, rice, corn, soybeans, wild oats, cocklebur, morningglory, johnsongrass, and barnyardgrass. The two pans were sprayed preemergence with the chemicals dissolved in a non-phytotoxic solvent.

Treated plants and controls were maintained in the greenhouse for 28 days, then all treated plants were compared to controls and visually rated for plant response.

Response ratings are based on a scale of 0 to 100 where 0 = no effect, and 100 = complete control. A dash (-) response means no test.

Response ratings are contained in Table 2. Data are combined from two tests.

Table 2 Results of Test B Compound A

| Rate g/ha | Postemergence | | | | | Preemergence | | | |
|---|---|---|---|---|---|---|---|---|---|
| | 1[1] | 4 | 16 | 62 | 250 | 4 | 16 | 62 | 250 |
| Corn | 90 | 95 | 100 | 100 | 100 | 0 | 40 | 80 | 100 |
| Wheat | 30 | 90 | 100 | 100 | 100 | 0 | 30 | 80 | 100 |
| Rice | 20 | 85 | 100 | 100 | 100 | 50 | 100 | 100 | 100 |
| Soybean | 30 | 85 | 65 | 100 | 100 | 0 | 20 | 55 | 93 |
| Cotton | 0 | 35 | 75 | 95 | 100 | 0 | 0 | 30 | 75 |
| Sugarbeet | 0 | 90 | 100 | 100 | 100 | 10 | 40 | 90 | 100 |
| Rapeseed | 0 | 0 | 0 | 15 | 50 | 0 | 0 | 0 | 10 |
| Crabgrass | 20 | 70 | 90 | 100 | 100 | 15 | 65 | 90 | 100 |
| Johnsongrass | 80 | 75 | 100 | 100 | 100 | 60 | 90 | 100 | 100 |
| Blackgrass | 0 | 75 | 90 | 100 | 100 | 40 | 95 | 100 | 100 |
| Barnyardgrass | 0 | 20 | 80 | 90 | 100 | 10 | 55 | 90 | 100 |
| Nutsedge | 0 | 0 | 30 | 40 | 85 | 0 | 15 | 35 | 80 |
| Giant Foxtail | 0 | 40 | 80 | 100 | 100 | 40 | 90 | 100 | 100 |
| Wild Oats | 0 | 25 | 55 | 90 | 100 | 25 | 55 | 90 | 100 |
| Cocklebur | 0 | 30 | 80 | 95 | 100 | 0 | 20 | 55 | 90 |
| Morningglory | 20 | 55 | 90 | 100 | 100 | 0 | 10 | 30 | 30 |
| Teaweed | 0 | 55 | 75 | 95 | 100 | 0 | 30 | 75 | 95 |
| Sicklepod | 0 | 30 | 65 | 100 | 100 | 0 | 10 | 50 | 85 |
| Jimsonweed | 20 | 85 | 95 | 100 | 100 | 0 | 25 | 80 | 100 |
| Velvetleaf | 50 | 85 | 95 | 100 | 100 | 0 | 25 | 80 | 100 |

[1] Rates = grams per hectare

Table 2 (continued) Compound B

| Rate g/ha | Postemergence 62 | 16 | 4 | 1 | Preemergence 250 | 62 | 16 | 4 |
|---|---|---|---|---|---|---|---|---|
| Corn | 100 | 100 | 90 | 70 | 100 | 90 | 40 | 30 |
| Wheat | 90 | 90 | 60 | 40 | 90 | 70 | 40 | 0 |
| Rice | 100 | 100 | 70 | 30 | 100 | 100 | 90 | 60 |
| Soybean | 100 | 100 | 70 | 60 | 100 | 90 | 40 | 0 |
| Cotton | 90 | 70 | 50 | 30 | 90 | 0 | 0 | 0 |
| Sugarbeet | 100 | 100 | 100 | 50 | 100 | 100 | 100 | 60 |
| Rapeseed | 0 | 0 | 0 | 0 | 70 | 30 | 0 | 0 |
| Crabgrass | 100 | 70 | 50 | 30 | 100 | 100 | 50 | 30 |
| Johnsongrass | 100 | 100 | 70 | 30 | 100 | 90 | 70 | 40 |
| Blackgrass | 100 | 100 | 50 | 30 | 100 | 100 | 90 | 70 |
| Barnyardgrass | 90 | 60 | 0 | 0 | 100 | 90 | 60 | 30 |
| Nutsedge | 90 | 70 | 30 | 30 | 100 | 60 | 30 | 0 |
| Giant Foxtail | 100 | 70 | 50 | 30 | 100 | 100 | 70 | 30 |
| Wild Oats | 90 | 80 | 30 | 0 | 90 | 70 | 40 | 0 |
| Cocklebur | 100 | 50 | 30 | 0 | 100 | 50 | 30 | 0 |
| Morningglory | 100 | 80 | 70 | 60 | 100 | 40 | 0 | 0 |
| Teaweed | 100 | 70 | 30 | 0 | 100 | 80 | 60 | 30 |
| Sicklepod | 100 | 100 | 60 | 30 | 100 | 70 | 50 | 30 |
| Jimsonweed | 100 | 100 | 70 | 40 | 100 | 90 | 60 | 30 |
| Velvetleaf | 100 | 100 | 70 | 80 | 100 | 90 | 60 | 30 |

Table 2 (continued) Compound C

| Rate g/ha | Postemergence 62 | 16 | 4 | Preemergence 250 | 62 | 16 |
|---|---|---|---|---|---|---|
| Corn | 90 | 90 | 80 | 80 | 20 | 0 |
| Wheat | 0 | 0 | 0 | 0 | 0 | 0 |
| Rice | 100 | 80 | 30 | 90 | 80 | 20 |
| Soybean | 20 | 20 | 0 | 30 | 0 | 0 |
| Cotton | 0 | 0 | 0 | 20 | 0 | 0 |
| Sugarbeet | 50 | 30 | 0 | 100 | 20 | 0 |
| Rapeseed | 0 | 0 | 0 | 20 | 0 | — |
| Crabgrass | 20 | 0 | 0 | 70 | 20 | 0 |
| Johnsongrass | 100 | 90 | 50 | 90 | 70 | 20 |
| Blackgrass | 60 | 50 | 0 | 80 | 50 | 0 |
| Barnyardgrass | 60 | 40 | 0 | 80 | 20 | 0 |
| Nutsedge | 0 | 0 | 0 | 0 | 0 | 0 |
| Giant Foxtail | 70 | 60 | 0 | 90 | 50 | 20 |
| Wild Oats | 0 | 0 | 0 | 60 | 20 | 0 |
| Cocklebur | 80 | 60 | 20 | 40 | 0 | 0 |
| Morningglory | 70 | 30 | 0 | 30 | 0 | 0 |
| Teaweed | 60 | 0 | 0 | 60 | — | 0 |
| Sicklepod | 80 | 50 | 0 | 30 | 0 | 0 |
| Jimsonweed | 90 | 80 | 50 | 90 | 50 | 0 |
| Velvetleaf | 60 | 30 | 20 | 60 | 0 | 0 |

## Claims

1. A compound of the formula:

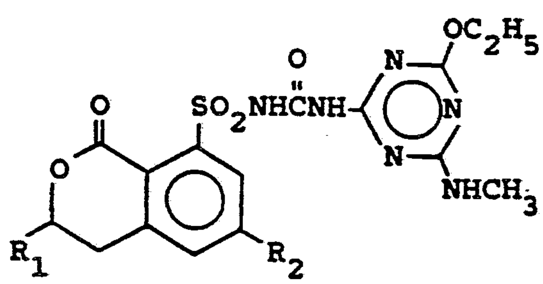

wherein

$R_1$ is H or $CH_3$; and

$R_2$ is H, F, Cl or $CH_3$.

2. A compound of Claim 1 which is N-[[4-ethoxy-6-(methylamino)-1,3,5-triazin-2-yl]aminocarbonyl]-3,4-dihydro-3-methyl-1-oxo-1H-2-benzopyran-8-sulfonamide.

3. A compound of Claim 1 which is N-[[4-ethoxy-6-(methylamino)-1,3,5-triazin-2-yl]aminocarbonyl]-3,4-dihydro-1-oxo-1H-2-benzopyran-8-sulfonamide.

4. A composition suitable for controlling the growth of undesired vegetation which comprises an effective amount of the compound of any of Claims 1 to 3 and at least one of the following: surfactant, solid or liquid diluent.

5. A herbicidal mixture comprising an effective amount of a compound of Claim 1 and an effective amount of a compound selected from:

sodium salt of 2-(1-allyloxy-aminobutylidene)-5,5-dimethyl-4-methoxycarbonylcyclohexane-1,3-dione;

N-ethyl-2-[[(phenylamino)carbonyl]oxy]propanamide (R)-isomer;

2,2-dichloropropionic acid;

S-(2,3-dichloroallyl)diisopropylthiocarbamate;

methyl 2-[4-(2',4'-dichlorophenoxy)phenoxy]-propanoate;

3-(3,4-dichlorophenyl)-1,1-dimethylurea;

(±)-butyl 2-[4-[(5-(trifluoromethyl)-2-pyridinyl)-oxy]phenoxy]-propanoate;

methyl 2-(4-((3-chloro-5-(trifluoromethyl)-2-pyridinyl)oxy)-phenoxy)propanoate;

2-chloro-N-(2,6-dimethylphenyl)-N-(1H-pyrazol-1-ylmethyl)-acetamide;

2-(α-naphthoxy)-N, N-diethyl propionamide;

4-methylsulfonyl-2,6-dinitro-N,N-dipropylaniline;

3,5-dichloro(N-1,1-dimethyl)-2-propynyl)benzamide;

2-[1-(ethoxyimino)butyl]-5-[2-(ethylthio)propyl]-3-hydroxy-2-cyclohexen-1-one;

trichloroacetic acid;

3,6-dichloro-2-pyridinecarboxylic acid;

α,α,α-trifluoro-2,6-dinitro-N, N-dipropyl-p-toluidine;

7-chloro-3-methylquinoline-8-carboxylic acid; and ethyl 2-[4-[(6-chloro-2-quinoxalinyl)oxy]phenoxy]propionate.

6. A method for controlling the growth of undesired vegetation which comprises applying to the locus to be protected an effective amount of a compound of any of claims 1 to 3.

7. A method for controlling the growth of undesired vegetation in rape which comprises applying to the locus to be protected an effective amount of a composition of Claim 5.

8. A method for controlling the growth of undesired vegetation in rape which comprises applying to the locus to be protected an effective amount of a compound of any of Claims 1 to 3.

9. A method for controlling the growth of undesired vegetation in rape which comprises applying to the locus to be protected an effective amount of a composition of Claim 5.

10. A process for the preparation of a compound of Claim 1 which comprises:

(a) reacting a sulfonylisocyanate of formula $JSO_2NCO$ with an appropriate heterocyclic amine of formula $NH_2$-A;

(b) reacting a sulfonylcarbamate of formula $JSO_2NHCO_2Ph$ with an appropriate heterocyclic amine of formula $NH_2$-A; or

(c) reacting sulfonamide of formula $JSO_2NH_2$ with an appropriate carbamate heterocycle of formula $C_6H_50.CO.NHA$;

wherein J is

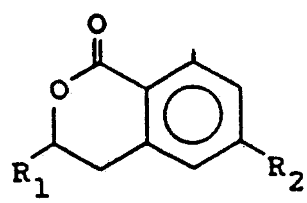

and A is

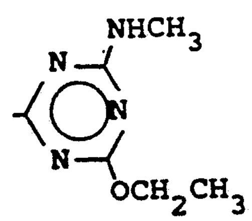

Claims for the contracting state: AT

1. A process for the preparation of a compound of the formula:

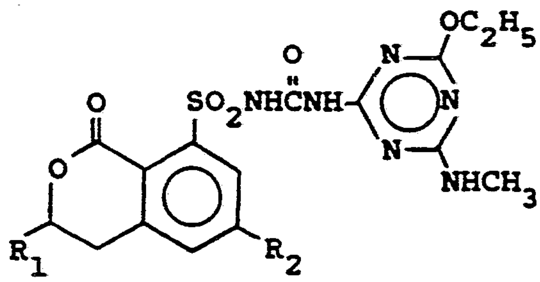

(I)

wherein

$R_1$ is H or $CH_3$; and

$R_2$ is H, F, Cl or $CH_3$;

which comprises

(a) reacting a sulfonylisocyanate of formula $JSO_2NCO$ with an appropriate heterocyclic amine of formula $NH_2$-A;

(b) reacting a sulfonylcarbamate of formula $JSO_2NHCO_2Ph$ with an appropriate heterocyclic amine of formula $NH_2$-A; or

(c) reacting a sulfonamide of formula $JSO_2NH_2$ with an appropriate carbamate heterocycyle of formula $C_6H_50.CO.NHA$; wherein J is

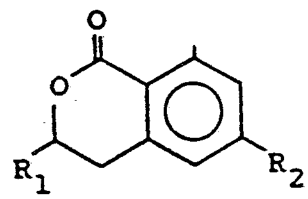

and A is

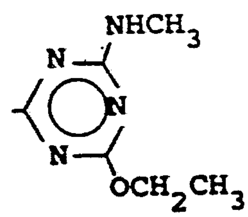

2. A process of Claim 1 wherein the product is N-[[4-ethoxy-6-(methylamino)-1,3,5-triazin-2-yl]aminocarbonyl]-3,4-dihydro-3-methyl-1-oxo-1H-2-benzopyran-8-sulfonamide.

3. A process of Claim 1 wherein the product is N-[[4-ethoxy-6-(methylamino)-1,3,5-triazin-2-yl]aminocarbonyl]-3,4-dihydro-1-oxo-1H-2-benzopyran-8-sulfonamide.

4. A composition suitable for controlling the growth of undesired vegetation which comprises an effective amount of a compound of formula (I) as defined in any of Claims 1 to 3 and at least one of the following: surfactant, solid or liquid diluent.

5. A herbicidal mixture comprising an effective amount of a compound of formula (I) as defined in Claim 1 and an effective amount of a compound selected from:

sodium salt of 2-(1-allyoxy-aminobutylidene)-5,5-dimethyl-4-methoxycarbonylcyclohexane-1,3-dione;

N-ethyl-2-[[(phenylamino)carbonyl]oxy]propanamide (R)-isomer;

2,2-dichloropropionic acid;

S-(2,3-dichloroallyl)diisopropythiocarbamate;

methyl 2-[4-(2',4'-dichlorophenoxy)phenoxy]-propanoate;

3-(3,4-dichlorophenyl)-1,1-dimethylurea;

(±)-butyl 2-[4-[(5-(trifluoromethyl)-2-pyridinyl)-oxy]phenoxy]-propanoate;

methyl 2-(4-((3-chloro-5-(trifluoromethyl)-2-pyridinyl)oxy)-phenoxy)propanoate;

2-chloro-N-(2,6-dimethylphenyl)-N-(1H-pyrazol-1-yl-methyl)-acetamide;

2-(α-naphthoxy)-N, N-diethyl propionamide;

4-methylsulfonyl-2,6-dinitro-N,N-dipropylaniline;

3,5-dichloro(N-1,1-dimethyl)-2-propynyl)benzamide;

2-[1-(ethoxyimino)butyl]-5-[2-(ethylthio)propyl]-3-hydroxy-2-

cyclohexen-1-one;

trichloroacetic acid;

3,6-dichloro-2-pyridinecarboxylic acid;

α,α,α-trifluoro-2,6-dinitro-N,N-dipropyl-p-toluidine;

7-chloro-3-methylquinoline-8-carboxylic acid; and

ethyl 2-[4-[(6-chloro-2-quinoxalinyl)oxy]phenoxy]proprionate.

6. A method for controlling the growth of undesired vegetation which comprises applying to the locus to be protected an effective amount of a compound of formula (I) as defined in any of claims 1 to 3.

7. A method for controlling the growth of undesired vegetation which comprises applying to the locus to be protected an effective amount of a composition of Claim 5.

8. A method for controlling the growth of undesired vegetation in rape which comprises applying to the locus to be protected an effective amount of a compound of formula (I) as defined in any of claims 1 to 3.

9. A method for controlling the growth of undesired vegetation in rape which comprises applying to the locus to be protected an effective amount of a composition of Claim 5.